# EUROPEAN PATENT APPLICATION

(11) **EP 1 475 041 A1**
(43) Date of publication of application: **10.11.2004**
(21) Application number: 04010209.7
(22) Date of filing: 29.04.2004
(51) Int. Cl.: A61B 17/00, A61M 25/10

(54) **Cardiac valve modification device**

(30) Priority: 30.04.2003 US 466940 P
(71) Applicant: Medtronic Vascular, Inc., Santa Rosa, CA 95403 (US)
(72) Inventor: Allen, Jeff W., Santa Rosa, CA 95405 (US); Brin, David S., Topsfield, MA 01983-1720 (US); Coppin, Chris M., Carlsbad, CA 92008 (US); Rafiee, Nasser, Andover,Massachusetts 01810 (US); Lamson, Theodore C., Pleasanton, CA 94566 (US)
(74) Representative: Bauer, Friedrich

(57) **Abstract**

The cardiac valve modification device of the present invention provides a catheter **22**; an injection assembly **24** disposed on the catheter **22**, the injection assembly **24** having lobes **26**, and a neck **28** disposed between the lobes **26**; and at least one injector **36** operably disposed at the neck **28**. The injector **36** can be an injection barb **60**, made of a biodegradable material including a therapeutic agent, and deposited in the valve annulus, or an injection needle **80**, which delivers a therapeutic agent to the valve annulus. The therapeutic agent can be a pro-fibrotic growth factor or a bulking agent. The cardiac valve modification method comprises inserting an injection catheter to the valve annulus (Block **142**); and injecting a therapeutic agent into the valve annulus with the injection catheter (Block **144**). The procedure can be repeated to modify the cardiac valve in a series of steps.

## Description

### RELATED APPLICATION

This application claims priority to U.S. Provisional Application No. 60/466,940, "Cardiac Valve Modification Method and Device" to Jeffrey W. Allen et al., filed April 30, 2003, the entirety of which is incorporated by reference.

### TECHNICAL FIELD

The technical field of this disclosure is medical devices and methods for treating cardiac valves, particularly, a method and device for modifying cardiac valves.

### BACKGROUND OF THE INVENTION

Human heart valves, such as the aortic and mitral valves are sometimes damaged by diseases or by aging which cause problems with the proper function of the leaflets and/or the sub-valvular apparatus attached to the leaflets. These valves consist of a valve annulus of fibrous tissue to which the flexible leaflets are attached. Often, degenerative disease causes the valve annulus to dilate to the point where the leaflets attached to it cannot fully close and allow valve leakage. This inability to close completely, a condition called valve insufficiency or incompetence, eventually requires surgical correction either by valve repair procedures or by valve replacement. In common practice, both repair and replacement require open-heart surgery with its attendant risks, expense, and extended recovery time.

Catheter based valve repair systems to modify the valve annulus using heat and radio frequency (RF) energized electrode catheters to shape the valve annulus or injected material are known in the art. The catheter based systems access the heart valve through a small incision in the groin or neck, which avoids opening the chest as in open-heart surgery. A heating device on the distal end of the catheter is applied to or inserted below the surface of the valve annulus and heat is applied to shrink and tighten the collagenous tissue of the valve annulus.

Use of RF energized electrode catheters has significant disadvantages. For example, often when using such a catheter to form a cardiac lesion, the cardiac tissue becomes charred from the RF energized heating of the tissue, blood near the cardiac tissue undergoing treatment becomes coagulated, and the cardiac tissue undergoes separation and/or popping.

U.S. Patent No. 6,267,781 to Tu discloses an ablation device for treating tissues, valvular annulus, valvular organ structure, or atherosclerosis of a patient, the ablation device including a flexible elongate tubular shaft having a deployable spiral wire electrode at its distal end adapted to contact/penetrate the tissue to be treated and to apply high frequency energy to the tissue for therapeutic purposes.

U.S. Patent No. 6,355,030 to Aldrich *et al*. discloses methods and devices for improving valve function in a heart, including a thermal heating device as part of a thermal heating member fixed to an elongate member, the thermal heating device being inserted into working space proximate the valve to be treated and being used to selectively contract the collagen fibers of the valve structure treated so as to improve the performance and functioning of the valve.

U.S. Patent No. 6,485,489 to Teirstein *et al.* discloses a delivery system and methods for repairing an annular organ structure comprising injecting a heat shapeable biomaterial formulated for in vivo administration by injection via a delivery system at a site of the valvular annulus defect; and applying heat sufficient to shape and immobilize the biomaterial at about the annulus defect, and optionally to shape tissue surrounding the annulus defect.

It would be desirable to have a cardiac valve modification method and device that would overcome the above disadvantages.

### SUMMARY OF THE INVENTION

One aspect of the present invention provides a cardiac valve modification device and method to change cardiac valve function by changing the flexibility and geometry of the valve annulus.

Another aspect of the present invention provides a cardiac valve modification device and method to inject a therapeutic agent into a valve annulus.

Another aspect of the present invention provides a cardiac valve modification device to locate an injection assembly at the valve annulus.

Another aspect of the present invention provides a cardiac valve modification method to gradually change cardiac valve function with a series of valve modifications.

The foregoing and other features and advantages of the invention will become further apparent from the following detailed description of the presently preferred embodiments, read in conjunction with the accompanying drawings. The detailed description and drawings are merely illustrative of the invention, rather than limiting the scope of the invention being defined by the appended claims and equivalents thereof.

### BRIEF DESCRIPTION OF THE DRAWINGS

**FIGS. 1-4C** show an injection catheter for cardiac valve modification made in accordance with the present invention.
**FIGS. 5 & 6** show another injection catheter for cardiac valve modification made in accordance with the present invention.
**FIG. 7** shows a flowchart for a method of cardiac valve modification in accordance with the present invention.
**FIG. 8** shows a flowchart for another embodiment of a method of cardiac valve modification in accordance with the present invention

### DETAILED DESCRIPTION

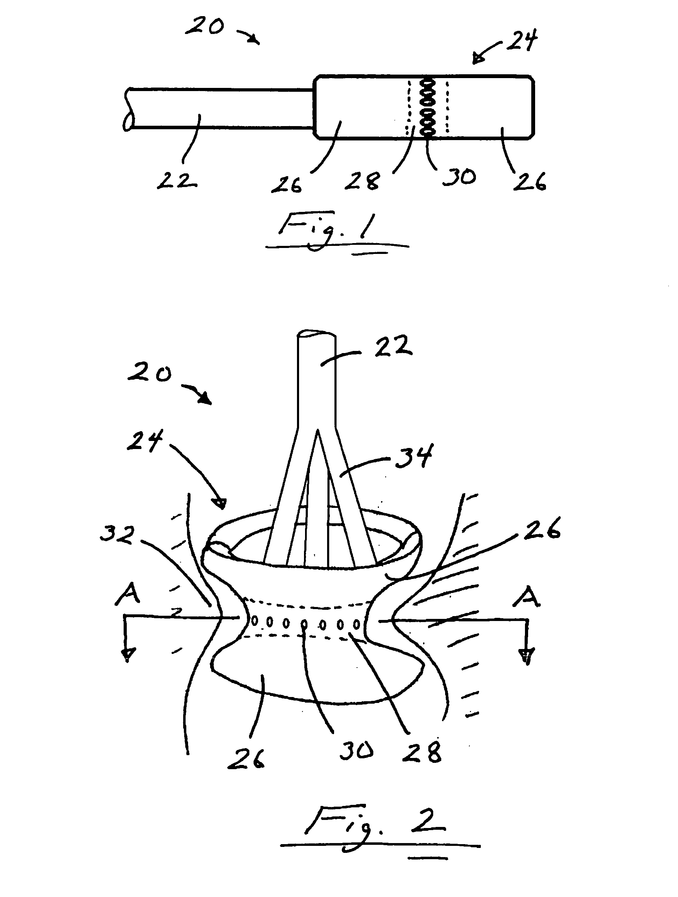

**FIGS. 1-4** show an injection catheter for cardiac valve modification. The injection catheter locates injectors in a cardiac valve annulus to dispense a therapeutic agent. The therapeutic agent may be, for example, a growth factor, a drug, a bulking agent or a biomaterial. In one embodiment, the therapeutic agent is a pro-fibrotic growth factor. The pro-fibrotic growth factor produces scarring in the valve annulus to improve cardiac valve function. In another embodiment, the therapeutic agent is a bulking agent such as collagen. Collagen injected into the valve annulus adds mass to the tissue surrounding the valve. The increased mass causes the valve leaflets to be drawn closer together reducing or eliminating valve leakage. In other embodiments the bulking agent may be, for example, a solidifying liquid or gel or a bead suspension.

**FIG. 1** shows an injection catheter in the deflated condition. The injection catheter **20** comprises a catheter **22** with an injection assembly **24** disposed on the distal end. The injection assembly **24** has lobes **26** and a neck **28** between the lobes. Injection ports **30** in the neck **28** provide openings for injectors. The catheter **22** includes lumens to inflate the lobes **26** and deploy the injectors. Inflatable portions of the injection assembly **24** can be made of a material such as polyethylene, polyethylene terephthalate (PET), nylon, PEBAX® polyether-block amide co-polymers, or the like.

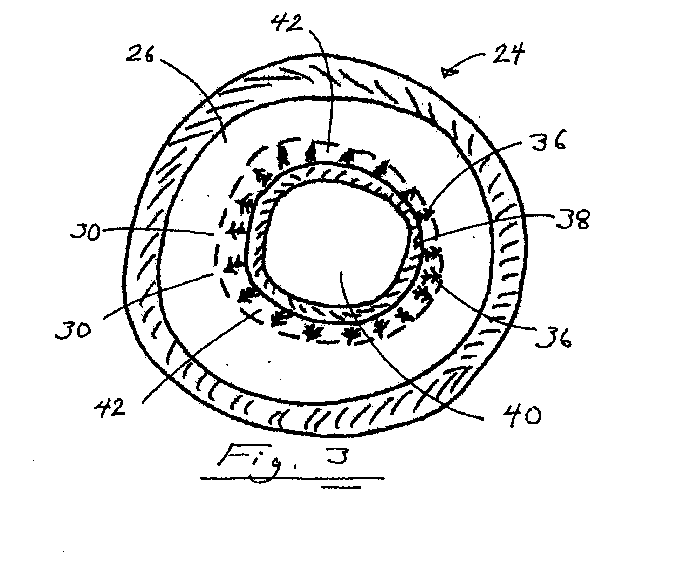

**FIG. 2**, in which like elements share like reference numbers with **FIG. 1**, shows an injection catheter in the inflated condition and disposed within a cardiac valve annulus. The inflated lobes **26** center the neck **28** on the cardiac valve annulus **32**, so that the injection ports **30** are located at the valve annulus **32**. This allows the injectors to be deployed into the valve annulus **32**. In one embodiment, the catheter **22** divides into catheter supports **34** to help stabilize the inflated injection assembly **24** within the valve annulus **32**. The catheter supports **34** can be connected to the inflatable lobes **26** and separate from each other as the lobes **26** inflate. In another embodiment, the catheter **22** can be a single, undivided shaft.

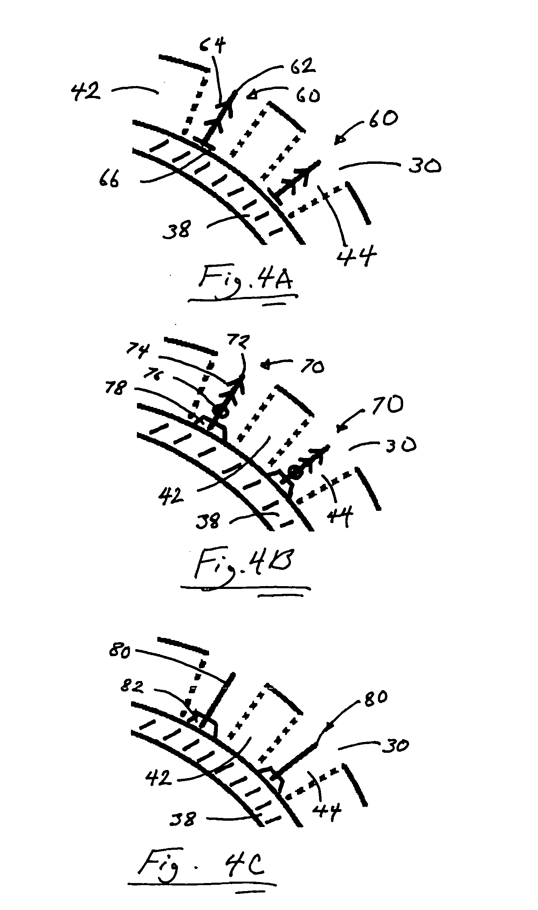

**FIG. 3** shows a cross section at A-A of **FIG. 2** of the injection catheter in the inflated condition. Injectors **36** are disposed in the injector space **42** behind the ports **30** outward of the inner balloon **38.** The inner balloon **38** is disposed in the neck. Inflating the inner balloon **38**, once the injection assembly **24** is in place within the valve annulus **32**, pushes the injectors **36** into the cardiac valve annulus. A lumen **40** permits blood flow through the injection assembly **24** during the valve modification procedure. In one embodiment, a check valve can be disposed in the lumen **40** to provide valve function during the valve modification procedure.

The injectors **36** can be injection barbs or injection needles. In one embodiment, the injectors **36** can be injection barbs including a therapeutic agent. The injection barbs remain in the valve annulus when the inner balloon **38** is deflated. In another embodiment, the injectors **36** can be injection needles dispensing a therapeutic agent. The therapeutic agent passes through the catheter and the injection needles into the valve annulus. The injection needles retract when the inner balloon **38** is deflated.

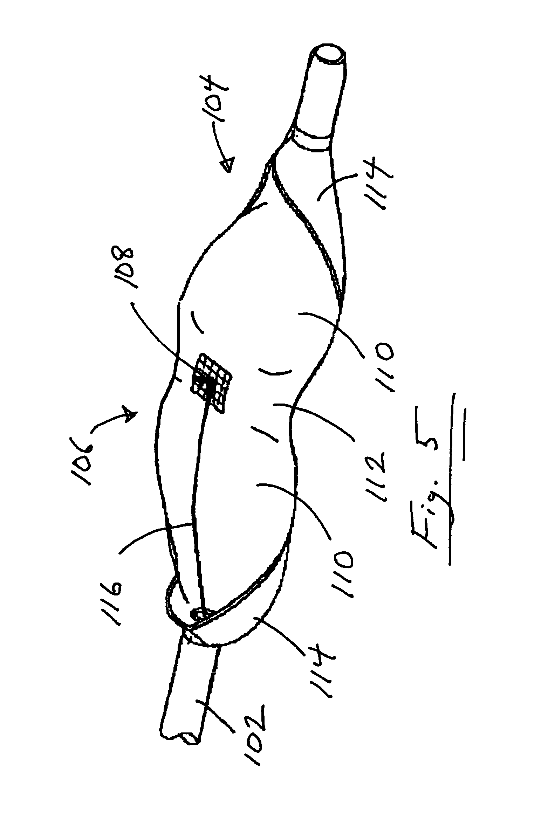

**FIGS. 4A-C** show detail of several embodiments of the injectors in the injection assembly. The injectors include a therapeutic agent for delivery into the cardiac valve annulus.

**FIG. 4A** shows a free standing injection barb **60** mounted in an injector bay **44** of the injection assembly. In this embodiment, the injection barb **60** is made of a biodegradable material including a therapeutic agent. The injection barb **60** is delivered into the valve annulus and remains there until the injection barb **60** is absorbed.

The injection barb **60** is disposed in injector bay **44** of the injector space **42** behind port **30.** The injection barb **60** comprises a pointed shaft **62**, barbs **64**, and a foot **66**. The foot **66** of the pointed shaft **62** is disposed against the inner balloon **38**, so that the inner balloon **38** can push the injection barb **60** from the injector bay **44** into the valve annulus when the inner balloon **38** is inflated. The barbs **64** maintain the injection barb **60** in the valve annulus on implantation. In one embodiment, the port **30** can be covered with a membrane to hold and protect the injection barb 60 as the injection assembly is advanced to the valve annulus. In another embodiment, the injector bay **44** can be filled with a dissolvable viscous material, such as a glutinous or gelatinous material, to enclose, hold, and protect the injection barb **60.**

The injection barb **60** can be made of a biodegradable material able to include a therapeutic agent, such as bioabsorbable polymers, including but not limited to polydioxanone, polyglycolic acid (PGA), polylactide (PLA), PGA/PLA copolymers, polycaprolactone, poly-b-hydroxybutyrate (PHB), combinations thereof, and the like. In one embodiment, the therapeutic agent can be any therapeutic agent able to produce scarring in the valve annulus, such as pro-fibrotic growth factor, including but not limited to transforming growth factor-β (TGF-β), connective tissue growth factor (CTGF), platelet derived growth factor (PDGF), insulin-like growth factor (IGF), and the like. The pro-fibrotic growth factors induce inflammation in the valve annulus, which heals to create scar tissue. The scar tissue improves the cardiac valve function by changing the flexibility and geometry of the valve annulus. The combination of the biodegradable material and the therapeutic agent can be tuned so that the therapeutic agent is released at a desired rate.

**FIG. 4B** shows an attached injection barb **70** mounted in an injector bay **44** of the injection assembly. In this embodiment, the injection barb **70** is made of a biodegradable material including a therapeutic agent. The injection barb **70** is delivered into the valve annulus and remains there until the injection barb **70** is absorbed.

The injection barb **70** is disposed in injector bay **44** of the injector space **42** behind port **30**. The injection barb **70** comprises a pointed shaft **72**, barbs **74**, a fracture point **76**, and an injector attachment **78**. The injector attachment **78** of the pointed shaft **72** is attached to the inner balloon **38**, so that the inner balloon **38** can push the injection barb **70** from the injector bay **44** into the valve annulus when the inner balloon **38** is inflated. The barbs **74** maintain the injection barb **70** in the valve annulus, so that the injection barb **70** breaks at the fracture point **76** and leaves the barbs **74** and most of the pointed shaft **72** in the valve annulus as the inner balloon **38** is deflated. The fracture point **76** can be a stress concentrator, such as a notch, or can be a weaker material than the rest of the pointed shaft **72**. The injection barb **70** can be made of a biodegradable material including a therapeutic agent as described for the injection barb of **FIG. 4A**.

Referring to **FIG. 4B**, the injection barb **70** can be unprotected or protected in the injector bay **44**. In one embodiment, the port **30** can be uncovered and the injector bay **44** unfilled. In another embodiment, the port **30** can be covered with a membrane to hold and protect the injection barb **70** as the injection assembly is advanced to the valve annulus. In another embodiment, the injector bay **44** can be filled with a dissolvable gel-like material to hold and protect the injection barb **70.**

**FIG. 4C** shows an attached injection needle **80** mounted in an injector bay **44** of the injection assembly. In this embodiment, the injection needle **80** delivers a therapeutic agent into the valve annulus and is then retracted and removed.

The injection needle **80** is disposed in injector bay **44** of the injector space **42** behind port **30**. The injection needle **80** includes a lumen (not shown) and is attached to the inner balloon **38** with an injector attachment **82**. The injector attachment **82** can be glue or a mechanical attachment, such as a rivet. The injector attachment **82** pushes the tip of the injection needle **80** from the injector bay **44** into the valve annulus when the inner balloon **38** is inflated. The tip of the injection needle **80** retracts when the inner balloon **38** is deflated. The injection needle **80** can be made of a sharpenable material, such as stainless steel, nitinol, or cobalt based alloys, such as MP35N or L605.

The lumen of the injection needle **80** communicates through the catheter to a therapeutic agent supply outside the patient. The therapeutic agent is injected at the cardiac valve annulus through the injection needle **80**. In one embodiment, the therapeutic agent can be any therapeutic agent able to produce scarring in the valve annulus, such as pro-fibrotic growth factor, including but not limited to transforming growth factor-β (TGF-β), connective tissue growth factor (CTGF), platelet derived growth factor (PDGF), insulin-like growth factor (IGF), and the like. The therapeutic agent can be included in microspheres for timed and coordinated release of one or more therapeutic agents. The pro-fibrotic growth factors induce inflammation in the valve annulus, which heals to create scar tissue. The scar tissue improves the cardiac valve function by changing the flexibility and geometry of the valve annulus.

In another embodiment, the therapeutic agent is a bulking agent. The bulking agent may be any therapeutic agent that adds mass to the tissue surrounding the valve when the agent is injected into or adjacent the valve annulus. For example, the bulking agent may be collagen, a bead suspension, a solidifying liquid or gel or an inflammatory agent.

Those skilled in the art will appreciate that many types of injection catheters can be used to deliver a therapeutic agent, such as pro-fibrotic growth factor, to the valve annulus. The injection catheter can have multiple needles injecting at multiple sites in the valve annulus at once, or can have a single needle and the injection catheter can be rotated to inject at multiple sites. In one embodiment, the injection catheter includes a plurality of needles positioned to inject a therapeutic agent into the annulus adjacent the anterior leaflet of the mitral valve. In this embodiment, the therapeutic agent is a bulking agent such as collagen or a bead suspension. An exemplary injection catheter with multiple needles is described in U.S. Patent No. 6,485,489 to Teirstein *et al.,* incorporated herein by reference. The injection catheter can be steerable to precisely locate the injection assembly at the valve annulus. Examples of such catheters include the MyoCath™ catheter from Bioheart, Inc., the MyoStar catheter from Johnson & Johnson, Inc., and the Stiletto catheter from Boston Scientific, Inc.

The location of the injection catheter relative to the valve annulus can be determined using an imaging or navigation system. In one embodiment, the distal tip of the injection catheter can have a radiopaque marker and fluoroscopy can be used to locate the distal tip. In another embodiment, a non-fluoroscopic navigation system, such as the Localisa® intracardiac navigation system from Medtronic, Inc., of Minneapolis, Minnesota, can be used to locate the distal tip. The Localisa® intracardiac navigation system uses three skin electrode pairs, positioned in x,y,z directions around the heart to track catheters. In yet another embodiment, fluoroscopy can be used in conjunction with a non-fluoroscopic navigation system to locate the distal tip.

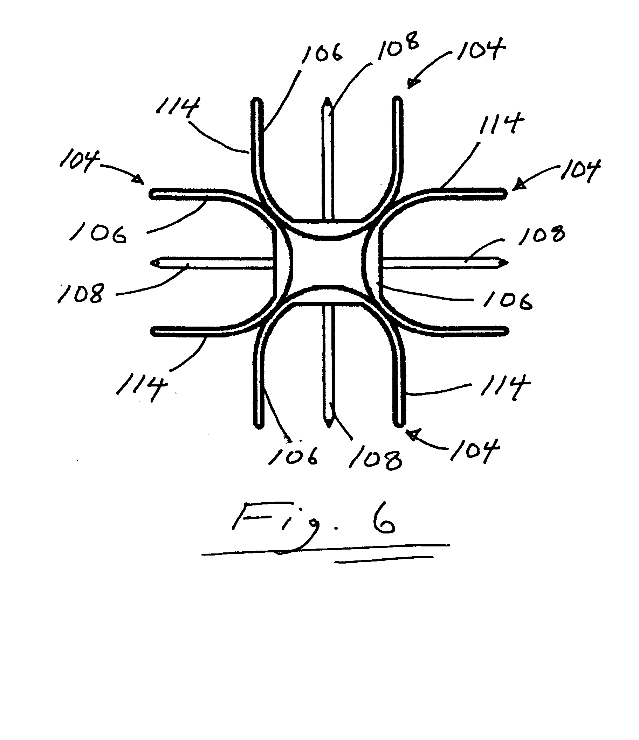

**FIGS. 5 & 6** show another injection catheter for cardiac valve modification. The injection catheter uses an injection assembly having at least one balloon with a needle disposed on the balloon. The balloon is inflated to deploy the needle to inject a therapeutic agent, such as pro-fibrotic growth factor or a bulking agent. The basic operation of an injection assembly of the present embodiment is described in U.S. Publication No. 2003/0055400 A1 and U.S. Patent No. 6,547,803 to Seward, *et al*., incorporated herein by reference.

**FIG. 5** shows an injection catheter in the inflated condition. The injection catheter **100** comprises a catheter **102** with an injection assembly **104** disposed on the distal end. The injection assembly **104** comprises a balloon **106** carrying a needle **108** and an actuator body **114**, the balloon **106** being furlable to fit within the actuator body **114** and withdraw the needle **108** in the deflated condition. The balloon **106** comprises lobes **110** and a neck **112** located between the lobes **110.** The needle **108** is attached at the neck **112,** so that the lobes **110** locate the needle **108** at the cardiac valve annulus when the balloon **106** is inflated. The neck **112** can be created on the balloon **106** by the method of manufacture or varying the materials at the neck **112.** A therapeutic agent, such as pro-fibrotic growth factor, can be injected through the needle **108** of the injector by means of the fluid line **116** communicating outside the patient through the catheter **102**. The pro-fibrotic growth factor creates scar tissue to improve cardiac valve function by changing the flexibility and geometry of the valve annulus.

**FIG. 6**, in which like elements share like reference numbers with **FIG. 5**, shows a cross section of a multi-needle injection catheter in the deflated condition. In this embodiment, several injection assemblies **104** are connected with the needles **108** pointing out from the center to form a multi-needle injection catheter. **FIG. 6** shows the cross section at the needles **108,** which are attached to the necks of the balloons **106.** When deployed, the balloons **106** extend the needles **108** from the actuator bodies **114**. In the inflated condition, the combined necks of the several injection assemblies **104** are smaller than the combined lobes, so that the necks locate the needles **108** at the smaller valve annulus. The actuator bodies **114** can be attached with glue or mechanical fasteners, or can be manufactured as an integral unit.

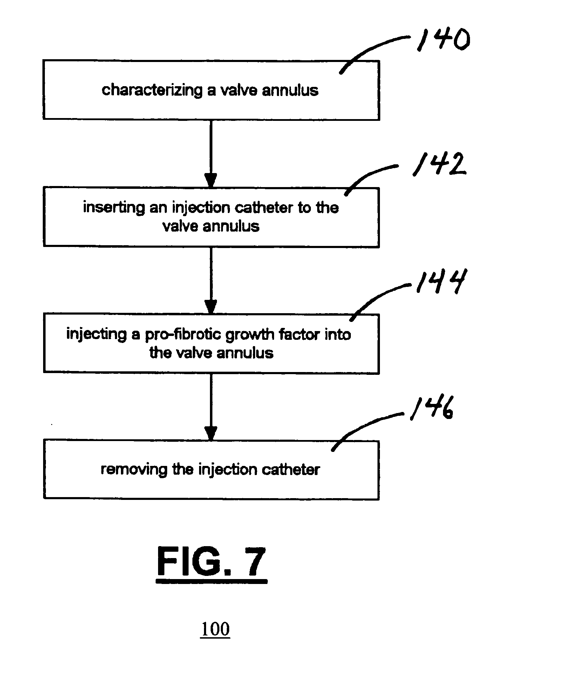

**FIG. 7** shows a flowchart for a method 100 of cardiac valve modification. A valve annulus is characterized (Block **140).** An injection catheter is inserted to the valve annulus (Block **142**) and a pro-fibrotic growth factor injected into the valve annulus with the injection catheter (Block **144**). The injection catheter is removed (Block **146**).

Characterizing the valve annulus (Block **140**) can comprise identifying target sites in the valve annulus where injection of a pro-fibrotic growth factor would improve valve function. The pro-fibrotic growth factor changes the flexibility and geometry of the valve annulus. Characterizing the valve annulus (Block **140**) can be performed by echocardiography, using echocardiography to identify valve leakage. In other embodiments, magnetic resonance imaging (MRI) or ultrafast computed tomography (CT) can be used to characterize the valve annulus.

While the injection catheter is transvascularly inserted to the valve annulus (Block **142),** the injection catheter can be located by an imaging or navigation system, such as fluoroscopy or a Localisa® non-fluoroscopic intracardiac navigation system from Medtronic, Inc. The injection catheter can be single needle or multi-needle, and can be steerable. Inserting the injection catheter to the valve annulus (Block **142**) can also comprise locating an injection assembly at the valve annulus, or deploying an injection assembly at the valve annulus, such as inflating an injection assembly.

Injecting pro-fibrotic growth factor into the valve annulus with the injection catheter (Block 144) can comprise injecting pro-fibrotic growth factor at target sites identified while characterizing the valve annulus. The pro-fibrotic growth factor can be a pro-fibrotic growth factor such as transforming growth factor-β (TGF-β), connective tissue growth factor (CTGF), platelet derived growth factor (PDGF), insulin-like growth factor (IGF), and the like. The pro-fibrotic growth factor can be included in microspheres for timed and coordinated release of one or more therapeutic agents.

In one embodiment, the method of cardiac valve modification can further comprise performing sensitivity testing to determine the proper pro-fibrotic growth factor and the sensitivity to particular pro-fibrotic growth factors for a particular patient. Different patients react differently, so sensitivity testing assures that the dose of a particular pro-fibrotic growth factor will achieve the desired amount of modification in the valve annulus. The sensitivity testing can be performed by injecting the patient subcutaneously with varied pro-fibrotic growth factors in varied strengths and measuring the inflammation and scarring reaction.

In another embodiment, the method of cardiac valve modification can further comprise performing a series of cardiac valve modification procedures, modifying the valve annulus in stages until the valve function is satisfactory. The inflammation and scarring process typically takes days to weeks after injecting pro-fibrotic growth factor, so the procedures can be performed weeks to months apart. The valve function can be checked after each procedure, using echocardiography or another similar method. Pro-fibrotic growth factor is injected into the valve annulus with each procedure if the valve function can be improved. The series approach assures that too great a modification is not applied in any one operation.

In another embodiment, the method of cardiac valve modification can further comprise checking the valve function after the cardiac valve modification procedure and repeating the cardiac valve modification procedure until valve function is satisfactory. The inflammation and scarring process typically takes days to weeks, so the checking of the results achieved is performed after that time. Checking the valve function can comprise checking the valve function using echocardiography. In other embodiments, magnetic resonance imaging (MRI) or ultrafast computed tomography (CT) can be used to check the valve function. If the further improvement in valve function is possible, the cardiac valve modification procedure can be repeated.

One method to monitor the progress of the inflammation and scarring process is to monitor C-reactive protein (CRP). CRP is an acute phase reactant released by the body in response to acute injury, infection, or other inflammatory stimuli. The inflammation in the valve annulus will release CRP. A decline in CRP following the cardiac valve modification procedure indicates the scarring is substantially complete. Monitoring the CRP will only be effective if no other acute injury, infection, or other inflammatory stimuli are present in the patient.

In another embodiment, the method of cardiac valve modification can further comprise modifying the scar tissue formed in the valve annulus by the cardiac valve modification procedure to further improve valve function. The scar tissue can be injected with a therapeutic agent, such as gluteraldehyde or other similar agents, to contract the scar tissue and further tighten the valve annulus. The therapeutic agent can be injected with an injection catheter as described herein for the injection of pro-fibrotic growth factors. Exposure to ultraviolet radiation can also be used to contract the scar tissue, by applying the ultraviolet radiation directly to the scar tissue.

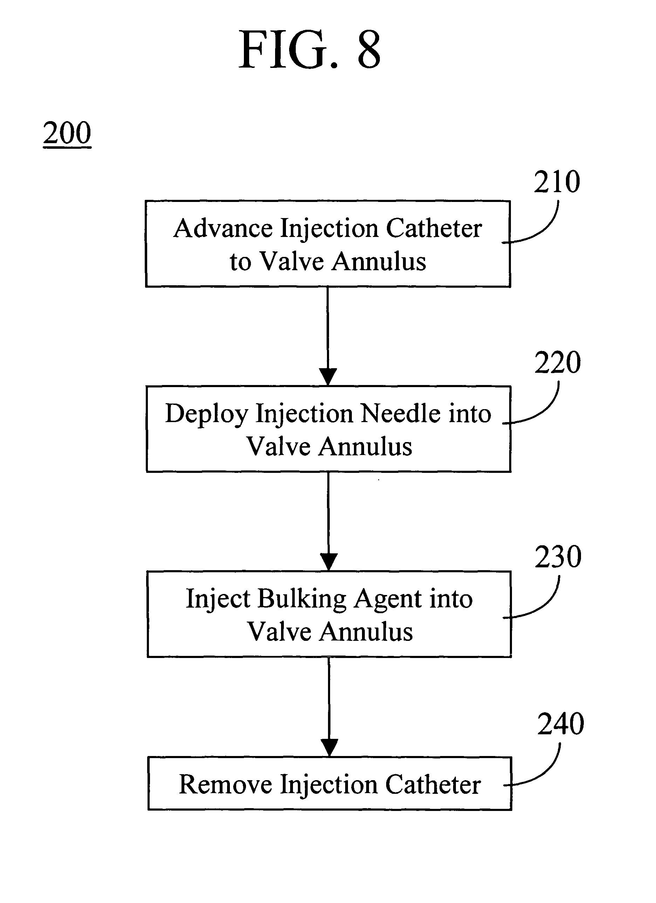

**FIG. 8** shows a flowchart for a method **200** of cardiac valve modification. Method 200 may utilize an injection catheter as described above in relation to FIGS. 1-6. Method 200 uses a bulking agent to modify the valve annulus. A distal end of an injection catheter is advanced via a transvascular (percutaneous) pathway to a position adjacent to the valve annulus (Block **210**). Once the distal end of the injection catheter is properly positioned, the injection assembly disposed on the distal end of the injection catheter is actuated to deploy the injection needle or needles (Block **220**).

The bulking agent is then injected into the valve annulus (Block **230**). The bulking agent may be any bulking agent or combination of bulking agents known to those with skill in the art. In one embodiment, the bulking agent is collagen. In another embodiment, the bulking agent is a suspension of beads. The injection catheter is removed after the bulking agent has been injected (Block **240**).

The bulking agent will modify the size and/or shape of the valve annulus by increasing the mass of the tissue surrounding the valve. The increase in tissue mass results in bringing the valve leaflets closer together, reducing or eliminating valve leakage.

In one embodiment, method **200** includes an injection assembly for treating a portion of a mitral valve annulus. In this embodiment, the injection assembly includes a plurality of needles positioned so that, when deployed, the needles are inserted only into the portion of the valve annulus adjacent the anterior leaflet of the valve. The bulking agent is then injected into the valve annulus only in the area adjacent the anterior leaflet. Injecting the bulking agent in this portion of the annulus is advantageous. This area of the mitral valve annulus is more fibrotic and dense as compared to the posterior side of the valve annulus providing a more stable region for pushing the valve leaflet into place.

It is important to note that **FIGS. 1-8** illustrate specific applications and embodiments of the present invention, and is not intended to limit the scope of the present disclosure or claims to that which is presented therein. Upon reading the specification and reviewing the drawings hereof, it will become immediately obvious to those skilled in the art that myriad other embodiments of the present invention are possible, and that such embodiments are contemplated and fall within the scope of the presently claimed invention.

While the embodiments of the invention disclosed herein are presently considered to be preferred, various changes and modifications can be made without departing from the spirit and scope of the invention. The scope of the invention is indicated in the appended claims, and all changes that come within the meaning and range of equivalents are intended to be embraced therein.

## Claims

1. A device for cardiac valve modification comprising:
a catheter **22**;
an injection assembly **24,104** disposed on the catheter **22**, the injection assembly **24, 104** having a first lobe **26, 110**, a second lobe **26, 110**, and a neck **28, 112** disposed between the first lobe **26, 110** and the second lobe **26, 110**; and
at least one injector **36** operably disposed at the neck **28, 112.**

2. The device of claim 1 wherein the first lobe **26** and the second lobe **26** are inflatable.

3. The device of claim 1 wherein the injector **36** is selected from the group consisting of an injection barb **60, 70** and an injection needle **80**.

4. The device of claim 1 wherein the neck **28** includes an inner balloon **38** and at least one port **30,** the injector **36** being disposed behind the port **30** and the inner balloon **38** being disposed behind the injector **36**.

5. The device of claim 4 wherein the port **30** is closed with a membrane.

6. The device of claim 4 wherein the injector **36** is enclosed in a viscous material.

7. The device of claim 4 wherein the injector 36 comprises a pointed shaft **72**, at least one barb **74** disposed on the pointed shaft **72**, an injector attachment **78**, and a fracture point **76** disposed between the barb **74** and the injector attachment **78**, the injector attachment **78** being attached to the inner balloon **38**.

8. The device of claim 4 wherein the injector 36 comprises an injection needle 80 attached to the inner balloon 38.

9. The device of claim 8 wherein the injection needle 80 includes a lumen for delivery of a therapeutic agent, the therapeutic agent selected from the group consisting of pro-fibrotic growth factor, transforming growth factor-β (TGF-β), connective tissue growth factor (CTGF), platelet derived growth factor (PDGF), insulin-like growth factor (IGF), and the like.

10. The device of claim 9 wherein the therapeutic agent is included in microspheres.

11. The device of claim 8 wherein the injection needle 80 includes a lumen for delivery of a bulking agent.

12. The device of claim 11 wherein the bulking agent is collagen.

13. The device of claim 1 wherein the injector 36 comprises a biodegradable material and a therapeutic agent.

14. The device of claim 13 wherein the biodegradable material is selected from the group consisting of bioabsorbable polymers, polydioxanone, polyglycolic acid (PGA), polylactide (PLA), PGA/PLA copolymers, polycaprolactone, poly-b-hydroxybutyrate (PHB), combinations thereof, and the like.

15. The device of claim 13 wherein the therapeutic agent is selected from the group consisting of pro-fibrotic growth factor, transforming growth factor-β (TGF-β), connective tissue growth factor (CTGF), platelet derived growth factor (PDGF), insulin-like growth factor (IGF), and the like.

16. The device of claim 1 wherein the injector 36 comprises a pointed shaft **62**, and at least one barb **64** disposed on the pointed shaft **62**.

17. The device of claim 16 wherein the injector **36** has a pointed end, and the injector **36** further comprises a foot **66** disposed on the pointed shaft **62** opposite the pointed end.

18. The device of claim 1 wherein the injection assembly **24** includes a lumen.

19. The device of claim 18 further comprising a check valve disposed in the lumen.

20. The device of claim 1 wherein the injection assembly **104** further comprises a balloon **106** and an actuator body **114**, the balloon **106** including the first lobe **110,** the second lobe **110,** and the neck **112** disposed between the first lobe **110** and the second lobe **110;** the injector **36** comprising a needle **108;** and the balloon **106** being furled within the actuator body **114** when the balloon **106** is deflated.

21. A system for cardiac valve modification comprising:
means for modifying a valve annulus;
means for injecting the modifying means into the valve annulus; and
means for locating the injecting means at the valve annulus.

22. The system of claim 21 wherein the modifying means is a scarring means selected from the group consisting of pro-fibrotic growth factor, transforming growth factor-β (TGF-β), connective tissue growth factor (CTGF), platelet derived growth factor (PDGF), insulin-like growth factor (IGF), and the like.

23. The system of claim 21 further comprising means for deploying the injecting means

24. The system of claim 21 further comprising means for tracking the injecting means.

25. The system of claim 22 further comprising means for testing patient sensitivity to the scarring means.

26. The system of claim 21 further comprising means for monitoring inflammation of the valve annulus.

27. The system of claim 22 wherein the scarring means produces scar tissue in the valve annulus and further comprising means for modifying the scar tissue.
